# EUROPEAN PATENT SPECIFICATION

(11) **EP 3 103 397 B1**
(45) Date of publication and mention of the grant of the patent: **28.10.2020**
(21) Application number: 16169575.4
(22) Date of filing: 30.03.2007
(51) Int. Cl.: A61B 10/02, A61B 10/00, A61B 17/00, A61B 90/30, A61B 50/30, A61B 90/00

(54) **TISSUE SAMPLE COLLECTION SYSTEM WITH VISUAL SAMPLE INSPECTION**
GEWEBEPROBENSAMMLUNGSSYSTEM MIT VISUELLER PROBENUNTERSUCHUNG
SYSTÈME DE PRÉLÈVEMENT D'ÉCHANTILLON DE TISSU AVEC INSPECTION VISUELLE DE L'ÉCHANTILLON

(30) Priority: 31.03.2006 US 788567 P
(43) Date of publication of application: 14.12.2016
(62) Divisional of application: 07722546.4
(73) Proprietor: Bard Peripheral Vascular Inc., Tempe, AZ 85281 (US)
(72) Inventor: ANDREASEN, Joergen, Tempe, AZ Arizona 85281 (US); VIDEBAEK, Karsten, Tempe, AZ Arizona 85281 (US); GUNDBERG, Tomas, Tempe, AZ Arizona 85281 (US); LARSEN, Lars Erup, Tempe, AZ Arizona 85281 (US)
(74) Representative: Hoffmann Eitle

(56) References cited:
- WO-A-96/24289
- WO-A-2007/021904
- DE-A1- 19 715 284
- US-A- 5 234 000
- US-A- 5 526 822
- US-A- 5 569 284

## Description

### Background of the invention

In modern medicine it is often desirable or necessary to harvest tissue samples from a target tissue region or tissue site Inside a human or animal body for the purpose of diagnosing a suspected malignancy.

Minimally invasive methods have partially replaced traditional surgical biopsies to minimize pain, discomfort and morbidity associated with surgical interventions. In the taking of biopsy tissue samples, it is frequently desirable to harvest multiple tissue samples in a single biopsy procedure to ensure that sufficient tissue from the target tissue region is available for analysis. Traditionally, such harvesting of multiple tissue samples required the physician or operator to make multiple passes with a needle-based device to acquire sufficient tissue. To solve this issue, prior art systems primarily adapted for breast biopsy applications provided the operator with the option of harvesting multiple tissue samples in a single device insertion. Such systems considerably ease the task of harvesting a larger number of samples, but they suffer from important limitations.

If it is desired that more than one biopsy sample is to be harvested in a single device insertion, the first tissue sample that is harvested will have to be removed from the sampling components of the biopsy device such as an tissue basket or container before more tissue samples can be harvested. Prior art biopsy devices have chosen between two basic tissue sample collection approaches; a manual and an automatic approach.

The manual systems provide the option of mechanically removing tissue samples from a tissue-receiving component with tweezers or similar means. The manual system enables the operator to visually inspect harvested tissue samples, and to maintain their relative orientation in the target region by placing the harvested tissue samples on strips of paper. This process is relatively slow and relies on human intervention which increases the risk of bio-contamination and other tissue sample degradation and errors.

US patent 5,526,822 discloses an automatic biopsy device capable of harvesting multiple samples from a target region inside a patient body and collect and individually place the harvested tissue samples in a flat substantially rectangular tissue sample cassette.

US patent 6,638,235 discloses another prior art automatic biopsy device.

Further, WO 96/24289 A2 teaches instruments for performing percutaneous biopsy procedures. These instruments comprise two types, single-use devices and multiple-use devices having active tissue capture capability.

US 5,526,822 B teaches a method and a device for automated biopsy and collection of soft-tissue having a piercing needle with a receiving port to trap tissue prior to cutting.

The automatic biopsy devices are often based on the application of vacuum suction to aspirate tissue samples from the point of sampling or target region into a storage container outside the body of the patient. This method permits the operator to concentrate on the task of harvesting multiple biopsies in a single device insertion without interruptions. However, the tissue sample storage may be more or less random and the storage container or cassette may become clogged, in effect spoiling the function.

### Description of the invention

It is an object of preferred embodiments of the present disclosure to at least partially overcome the drawbacks of the prior art devices known to the inventors. One object of preferred embodiments of the disclosure is to provide a new tissue-ejecting and tissue-collecting system. Another object of preferred embodiments of the disclosure is to provide a system, which facilitates visual inspection, and which reduces the risk of tissue-sample degradation.

The present invention is directed to the biopsy device of claim 1. Preferred embodiments are defined in the dependent claims.

In a first aspect, the disclosure provides a biopsy device for harvesting at least one tissue sample from a body of a living being, comprising:
- a hollow needle with a distal end portion adapted to be introduced into the body;
- a cutting mechanism for severing the at least one tissue sample;
- a sample-receiving device for receiving the at least one severed tissue sample, the sample- receiving device being receivable in the hollow needle and movable therein between a first extended position and a second retracted position;
- a tissue sample-collecting device comprising one or more tissue-retaining cavities;
- an ejector system for ejecting the tissue sample from the tissue sample-receiving device into one of said tissue-retaining cavities, when the sample-receiving device is in the second retracted position;
- an inspection system allowing an operator of the biopsy device to visually inspect the tissue sample ejected into said tissue-retaining cavity, while said hollow needle remains in the body.

In a second aspect, the disclosure in particular provides biopsy device for harvesting at least one tissue sample from a body of a living being, comprising:
- a hollow needle with a distal end portion adapted to be introduced into the body;
- a cutting mechanism for severing the at least one tissue sample;
- a sample-receiving device for receiving the at least one severed tissue sample, the sample- receiving device being receivable in the hollow needle and movable therein between a first extended position and a second retracted position;
- a tissue sample-collecting device comprising a rotatable drum having a plurality of tissue-retaining cavities arranged along its circumference;
- an ejector system for ejecting the tissue sample from the tissue sample-receiving device into one of said tissue-retaining cavities, when the sample-receiving device is in the second retracted position;
wherein the rotatable drum is rotatable relative to the sample-receiving device in such a way that different tissue samples harvested at different times can be accommodated in separate ones of said plurality of tissue-retaining cavities.

Embodiments of the disclosure are described below.

It will be appreciated that embodiments of the first aspect of the disclosure provide an automated biopsy device which comprises an optical inspection means for inspecting individual tissue samples, preferably immediately following their harvesting and transport out of the patient body. The automated biopsy device of the present invention is preferably capable of harvesting multiple tissue samples during a single needle insertion and may comprise a tissue sample cassette that supports automatic, systematic and reliable tissue sample collection. A preferred embodiment of the automated biopsy device may advantageously collect the harvested tissue samples in a manner that maintains the sequence, direction and orientation of the individual tissue samples.

A preferred embodiment of the automated biopsy device may also permit the operator to visually inspect each tissue sample immediately following its excision from the body of the patient without requiring the operator to remove the entire biopsy device from the body of the patient.

The visual inspection system of the present disclosure is inter alia advantageous where tissue samples are harvested from multiple locations such as in connection with biopsying of the prostate. Under these circumstances it is desirable that all tissue samples are adequate in the sense that they allow a reliable diagnosis to be made. Ensuring that harvested tissue samples are adequate before submitting these for pathologist analysis may reduce the number of patient revisits caused by inadequate biopsy procedures where insufficient or damaged tissue material was produced. Accordingly, the inclusion of suitable visual inspection means on a multiple automated biopsy device has the potential of significantly reducing patient anxiety, patient discomfort and medical costs to the healthcare system. The inspection system may comprise at least one of: an ocular, a periscopic cone, and a camera system.

An embodiment of an automated biopsy device comprising, e.g. a substantially cylindrical, rotating tissue-collecting cassette with a number of separate tissue-collecting chambers coupled to an ejector system that lift tissue samples out of a tissue-receiving basket or container into the tissue sample cassette while at the same time providing the operator with an active or passive optical inspection means for inspecting each tissue sample has been conceived as further described below.

One advantage of preferred embodiments of the present device over prior art is that it presents a tissue collection cassette with chambers that may be made of a transparent material and are semi-open, to provide easy insertion of one or more tissue samples into one or more cassette chambers while at the same time permitting visual inspection of individual tissue samples immediately following insertion of said tissue samples into said chambers.

According to one embodiment of the disclosure, the tissue sample cassette may be operatively coupled to a passive or active visual inspection means such as an ocular, a periscopic cone or a camera that may provide enhanced visual inspection of each harvested tissue sample subsequent to its collection. The visual inspection means enables the biopsy device operator to ensure tissue sample adequacy immediately following excision and address potential inadequacies by harvesting one or more additional tissue samples from the target region.

As an additional feature, the visual inspection means may provide a means of magnifying a harvested tissue sample for an enhanced more detailed inspection and evaluation.

The active visual inspection means may comprise a camera such as a digital camera, because digital cameras enable transmission or projection of tissue sample images onto screens for improved visualization of harvested tissue samples.

The tissue sample images may furthermore be transmitted live to one or more remote locations to permit more spectators to participate in a biopsy session, for instance during training sessions, medical conferences or during medical research. According to another embodiment of the present automated biopsy device, the tissue sample cassette incorporates light illuminating means such as one or more light sources selected from the group of LEDs, laser devices, light bulbs etc to illuminate the individually harvested tissue samples after collection to assist the operator during visual inspection of tissue samples.

An advantageous embodiment of the present biopsy device comprises means to support individual automatic and systematic collection of one or more tissue samples when they have been cut from the target region or site in the patient's body and subsequently transported out of the body of the patient. The tissue sample cassette is configured to maintain the axial and radial orientation of the individual tissue samples.

One embodiment of the disclosure comprises a tissue sample cassette or cassette that is removably attached to a disposable unit of the automated biopsy device and may be removed from the disposable unit following the biopsy procedure. As the harvested tissue samples are still held in their individual cassette compartments, the cassette may function as a tissue sample storage medium. For instance, the cassette may be inserted in a storage container that may be filled with formalin or a similar storage agent, whereupon tissue autolysis is halted and the tissue samples may be sent to the pathologist without further degradation.

Yet another embodiment of the disclosure comprises a cassette which includes metric marcs, to assist the operator in assessing tissue sample adequacy and the cassette may optionally be configured to additionally include numeric markings on each cassette chamber, to assist the operator and the pathologist in keeping track of harvested tissue samples.

As it will be apparent from the above description, the first and second aspects of the provide a biopsy device including an ejector system for ejecting the tissue sample from the tissue sample-receiving device into the tissue-retaining cavitie(s) of the tissue sample-collecting device. In one embodiment, the ejector system may be at least partially integrated in the tissue sample-collecting device and/or in the sample-receiving device, such that at least a part of the ejector system forms a portion of the sample-receiving device. For example, a wall or rim partition of the sample-collecting device may form a scooping element for scooping the tissue sample out of the sample-receiving device. Hence, a wall partition of a cavity of the sample-receiving device and said wall or rim partition of the sample-collecting device may together constitute the ejector system.

In one embodiment, the tissue sample-collecting device comprises a plurality of tissue-retaining cavities for receiving individual tissue samples, and the tissue sample-collecting device and the sample-receiving device are movable relative to each other in such a way that different tissue samples harvested at different times can be accommodated in seaparate ones of the plurality of cavities.

Optionally, each of the one or more tissue-retaining cavities may communicate with a mutual tissue-collecting chamber. In such embodiments, the tissue sample-collecting device and the ejector system may be operable to selectively
(a) collect a plurality of tissue samples in a plurality of separate tissue-retaining cavities, or
(b) collect a plurality of tissue samples in the mutual tissue-collecting chamber.

Hence, an operator of the device may chose to collect multiple samples in the mutual tissue-collecting chamber, if he Is not concerned about keeping the samples separate, i.e. if his main concern is to collect a relatively large volume of tissue. Alternatively, he may decide to collect individual samples in separate cavities, if it for instance is desirable to analyze the samples individually. Both options are available in the above-recited embodiment of the present disclosure. In one embodiment, the mutual tissue-collecting chamber is provided centrally with respect to circumferentially arranged cavities and/or scooping elements.

Generally, the sample-collecting device may e.g. comprise a rotatable drum, which comprises the plurality of cavities arranged along its circumference. In the present context, the term "drum" is to be understood as any rotatable element, including a cylindrical or conical element, or any other rotatable element. The outer surface of the drum may be smooth-walled, or it may feature a number of projections or depressions, such as elements for collecting and/or ejecting tissue from the sample-receiving device.

The drum may be rotatable around an axis of rotation, which is essentially parallel to a longitudinal axis of the hollow needle, or it may be rotatable around an axis of rotation, which extends transversely to a longitudinal axis of the hollow needle. In both cases, the drum may itself comprise the ejector system of part thereof as discussed previously.

The Inspection system of preferred embodiments of the present disclosure may be arranged such that it allows for inspection of a tissue sample after ejection of the tissue sample into one of said cavities and after relative movement of the sample-receiving device and the sample-collecting device to relatively displace said one cavity accommodating the tissue sample away from that position, in which the tissue sample has been ejected into said one cavity. Hence, collecting of a further sample may take place, while the previously collected sample is simultaneously being inspected.

The sample-collecting device is preferably comprised in a disposable unit of the biopsy device. Preferably, the disposable unit is releasably mountable in a reusable unit. Most preferably, the disposable unit includes those elements of the biopsy device, which come Into contact with body tissue, body fluid or other body matter, or which otherwise are exposed to contamination. The reusable unit preferably includes driving elements for the transport of the sample-receiving device between the first extended position and the second retracted position, battery cells, operator interface, control circuit, and/or other components which can be kept physically isolated from tissue, body fluid and other body matter, and which consequently may not be exposed to contamination, and which do not come into contact with the patient's tissue or body fluids.

The inspection system may be provided as a part of the disposable unit or as a part of the reusable unit. In case the inspection system includes costly optics and/or camera facilities, it is preferably included in the reusable unit. In some embodiments, the inspection system is only partially included in the biopsy device, with parts of the inspection system being provided remote from the biopsy device. For example, the biopsy device may include a camera and a wired or wireless signal transmission element for communicating an output signal of the cameral to a remote receiver, such as a monitor or a data-processing facility.

In one embodiment, the ejector system comprises a plurality of movable pins arranged with mutual spacings along a longitudinal axis of the hollow needle. The hollow needle may comprise a plurality of first openings facing the pins, and the sample-receiving device may comprise a plurality of second openings, which are aligned with the first openings, when the sample-receiving device is in the second retracted position, so that the pins can penetrate the first and second openings to eject the tissue sample. This setup has been found to be useful for ejecting samples of relatively firm tissue, e.g. cartilage or muscle tissue.

In an alternative embodiment, the tissue sample-collecting device comprises at least one scooping element arranged on its outer circumference. The scooping element may be arranged to collect tissue In the sample-receiving device, when the sample-receiving device is in its second retracted position or when the sample-receiving device is moving towards its second retracted position. Hence, the ejector system and sample-collecting device may be Integrated into one unit in the sense that the sample-collecting device with its at least one scooping element may co-operate with a wall partition of the sample-receiving device to constitute the ejector system.

The tissue sample-collecting device with the at least one scooping element may be movable between a first elevated position, in which the scooping element does not engage a tissue-receiving container of the sample-receiving device, and a second lowered position, in which said scooping element engages said tissue-receiving container. Hence, when the tissue sample-collecting device is in the first elevated position, the sample-receiving device is movable between the first extended and the second retracted positions. In order for the scooping element to collect tissue from the sample-receiving device, the scooping element may be brought to the second lowered position, in which it engages the tissue-receiving container. As an alternative to the scooping element being movable between an elevated and a lowered position, the sample-receiving device or at least a portion thereof may be movable relative to the tissue sample-collecting device with the at least one scooping element.

The at least one scooping element may be provided as a separate element, or it may form at least a wall of at least one of the tissue-retaining cavities.

It should be understood that the mutual tissue-collecting chamber discussed above is regarded as a separate disclosure.

Hence, in an independent aspect, the present disclosure also provides a biopsy device for harvesting at least one tissue sample from a body of a living being, comprising:
- a hollow needle with a distal end portion adapted to be introduced into the body;
- a cutting mechanism for severing the at least one tissue sample;
- a sample-receiving device for receiving the at least one severed tissue sample, the sample-receiving device being receivable in the hollow needle and movable therein between a first extended position and a second retracted position;
- a tissue sample-collecting device comprising one or more tissue-retaining cavities;
- an ejector system for ejecting the tissue sample from the tissue sample-receiving device into one of said tissue-retaining cavities, when the sample-receiving device is in the second retracted position;
wherein each of said one or more tissue-retaining cavities communicates with a mutual tissue-collecting chamber. The tissue sample-collecting device and the ejector system are preferably operable to selectively (a) collect a plurality of tissue samples in a plurality of separate tissue-retaining cavities, or (b) collect a plurality of tissue samples in the mutual tissue-collecting chamber.

In all aspects of the present disclosure, the ejector system may, as an alternative or in addition to the ejector systems described above, include a setup for ejecting tissue be liquid flushing, e.g. as disclosed in international patent publication No. WO : 2006/005345.

In order to move the sample-receiving device, e.g. to move the sample-receiving device in the hollow needle between the first extended and the second retracted positions, there may be provided a transport device comprising a bendable elongate element, e.g. as disclosed in international patent publication No. WO : 2006/005344.

To introduce the hollow needle and the sample-receiving device into suspect (target) tissue mass and to sever a tissue sample, there is preferably provided at least one firing mechanism for causing the hollow needle and the sample-receiving device to be longitudinally displaced in a distal direction, so as to penetrate body tissue at or near the suspect tissue mass. The same firing mechanism, or alternatively a further firing mechanism, may be provided for causing the hollow needle to be longitudinally displaced in a distal direction from a first position, in which the sample-receiving device projects from the distal end of the hollow needle, to a second position, in which the hollow needle essentially accommodates the cavity of the sample-receiving device, so as to sever said tissue sample from remaining body tissue at the harvesting site. For example, two user-operable firing mechanisms may be provided as disclosed In WO 2006/005342.

In preferred embodiments of the disclosure, the sample-receiving device and the hollow needle are comprised in a disposable unit, which is releasably attachable to a reusable unit including e.g. drive components for providing a driving force for transporting the sample-receiving device. In such embodiments, there is preferably provided a control system for controlling movement of the transport device and for arresting the sample-receiving device in the second retracted position, as disclosed in international patent publication No. WO 2006/005343. For example, the control system may be configured to automatically detect a distance between the first extended position and the second retracted position of the sample-receiving device, as disclosed in WO 2006/005343.

### Detailed description of embodiments of the disclosure

Embodiments of the disclosure will now be described further with reference to the accompanying drawings, in which:
Figs. 1-15 Illustrate a first embodiment of a tissue-collecting device and ejector system;
Figs. 16-31 illustrate a second embodiment of a tissue-collecting device and ejector system;
Figs. 32 and 33 generally illustrate an embodiment of a biopsy device according to the disclosure ;
Figs. 34-38 illustrate an embodiment of a transport mechanism and cutting mechanism.

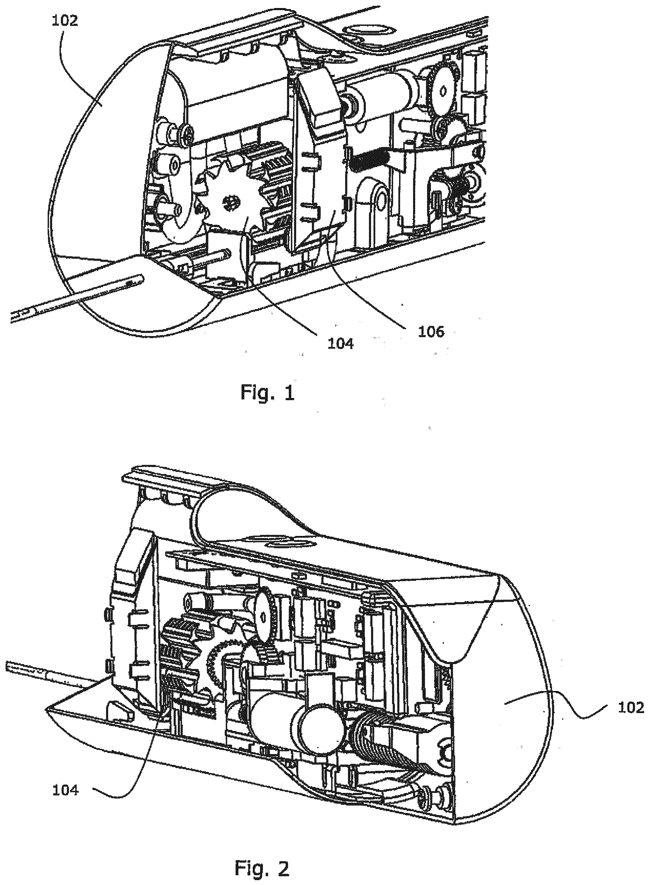

Figs. 1-3 show three 3D views of an embodiment of the biopsy device with the tissue sample cassette system and an optical cone placed adjacent to the cassette.

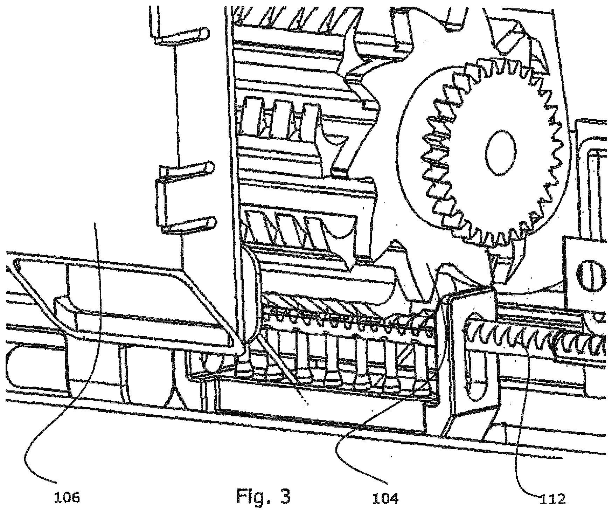

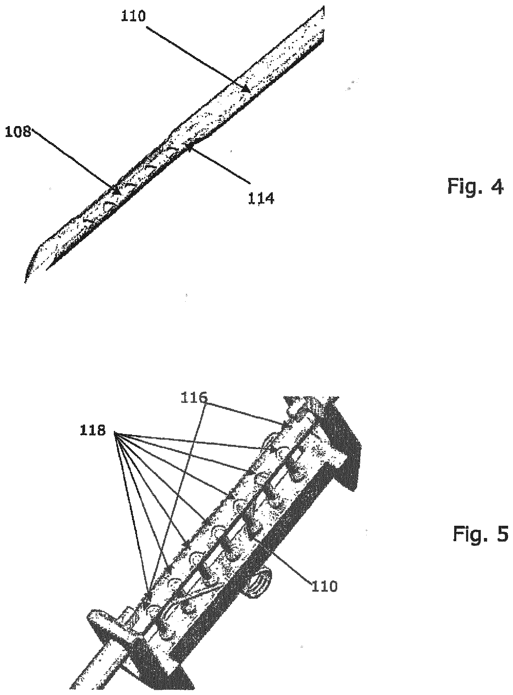

Fig. 1 is a semi-frontal view where a housing 102 of the biopsy device has been made transparent from the left side, showing a tissue-collecting cassette 104 behind an ocular 106. Fig. 2 is a semi-rearward view from the left, showing more clearly the shape of the cassette 104. Fig. 3 is a detail semi-rearward view of the cassette 104 from the left. The ejection zone where the cassette 104 meets a tissue-receiving basket or container and the placement of the ocular 106 relative to the cassette 104 are shown. Optical inspection of the tissue-sample takes place immediately following ejection of the tissue sample from the tissue-receiving container 108 (cf. Fig. 4).

One embodiment of the present biopsy device comprises a disposable unit comprising invasive components, and a non-disposable unit comprising the driver components. The biopsy device works by positioning a generally tubular tissue-receiving container 108 in a suspect tissue region. The tissue-receiving container 108 is movable between a first advanced and a second retracted position within the inner lumen of a retractable cutting cannula 110, cf. Fig. 4. The cutting cannula 110 and the tissue-receiving container 108 are placed in their first advanced positions during insertion into the anatomy of the patient.

Subsequent retraction of the cutting cannula 110 exposes a lateral opening in the tissue-receiving container that leads a cavity in the tissue-receiving container 108, and permits adjacent tissue to prolapse into the cavity of the tissue-receiving container. Vacuum, drawn through the inner lumen of the cutting cannula 110, further assists the prolapse of tissue. In the retracted position of the cutting cannula 110 where the lateral opening of the tissue-receiving container 108 is fully exposed and the cavity has been filled with tissue from the target region, the cutting cannula 110 is rapidly advanced and the prolapsed tissue is severed and captured, as the end portion of the cutting cannula forms a circumferential cutting edge. During a subsequent retraction of the tissue-receiving container 108 towards its second retracted position by means of a transport mechanism, the harvested tissue sample is moved out of the body of the patient.

In one preferred embodiment the transport mechanism is configured as a bendable toothed rack 112 (cf. Fig. 3) that interfaces with a number of cogs to provide linear motion of the tissue-receiving container 108. It is, however, to be understood that other flexible rods, racks or bands may substitute the toothed rack 112. When the tissue sample has been moved fully out of the body of the patient, it may be ejected from the cavity of the tissue-receiving container 108 and collected in the tissue-collecting cassette 104. The tissue-receiving container 108 may then be repositioned in the suspect tissue region by means of the toothed rack 112, and a new tissue sample may be harvested by repeating the procedure as outlined above.

As shown in Fig. 4, the present embodiment of the biopsy device comprises an outer cutting cannula 110 (or hollow needle) with a proximal end and a distal, sharpened end 114, including the circumferential cutting edge, as well as the flexible toothed rack 112 (cf. Fig. 3) that is received in the inner lumen of the cutting cannula and is longitudinally movable along the axis of the outer cutting cannula between a first advanced and a second retracted position. The tissue-receiving container 108 in Fig. 4 is herein also referred to as a sample-receiving device.

The toothed rack 112 is operatively connected to the hollow tubular tissue-receiving container 108 comprising a container cavity and an elongate opening in the topmost part. Said container is arranged in continuation of the distal end of the toothed rack 112.

Referring to Fig. 5, an ejector opening 116 in the proximal part of the outer cutting cannula 110 has a length that corresponds with the length of the opening in the tissue-receiving container 108 and permits access to the container cavity. The second retracted position of the tissue-receiving container corresponds with this opening, and the tissue sample may thus be collected from the cavity of the tissue-receiving container 108 through the ejector opening 116. In the bottom portion of the cutting cannula 110, and directly opposite the ejector opening 116, an axially aligned row of ejector pin holes 118 is provided. These holes correspond with an equal number of axially aligned ejector pin holes in the bottom of the tissue-receiving container 108, and when the tissue-receiving container is in its second retracted position, the two sets of holes are vertically aligned.

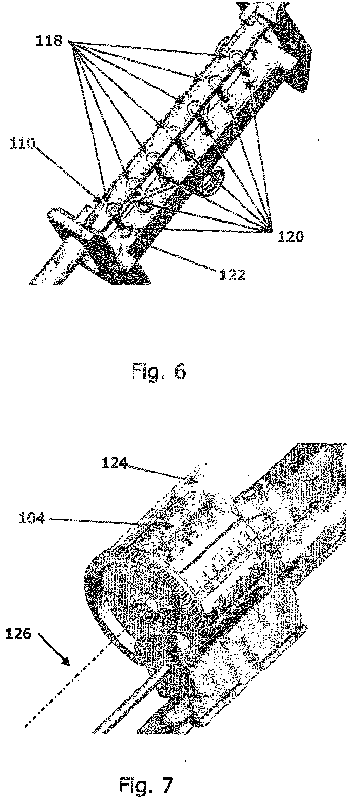

As shown in Fig. 6, the ejector pin holes in the cutting cannula 110 and the tissue-receiving container 108 are configured to receive a number of vertically movable ejector pins 120 that are mounted on an ejector frame 122 and are introduced into the tissue-receiving container 108 from a point below the bottom of the cutting cannula 110. The ejector pins 120 push tissue kept in the container through both the elongate opening of the tissue-receiving container and the ejector opening in the cutting cannula. When tissue has been pushed all the way through the ejector opening, it may be collected by a suitable means of collection.

As shown in Fig. 7, the cylindrical, elongate tissue sample cassette (rotatable drum) 104 comprises such a means of collection. It is removably placed in a cassette housing 124 that is removably attached to a disposable part of the biopsy device, and is stepwise rotatable about a central axis 126 that is parallel to the axis of the outer cutting cannula 110.

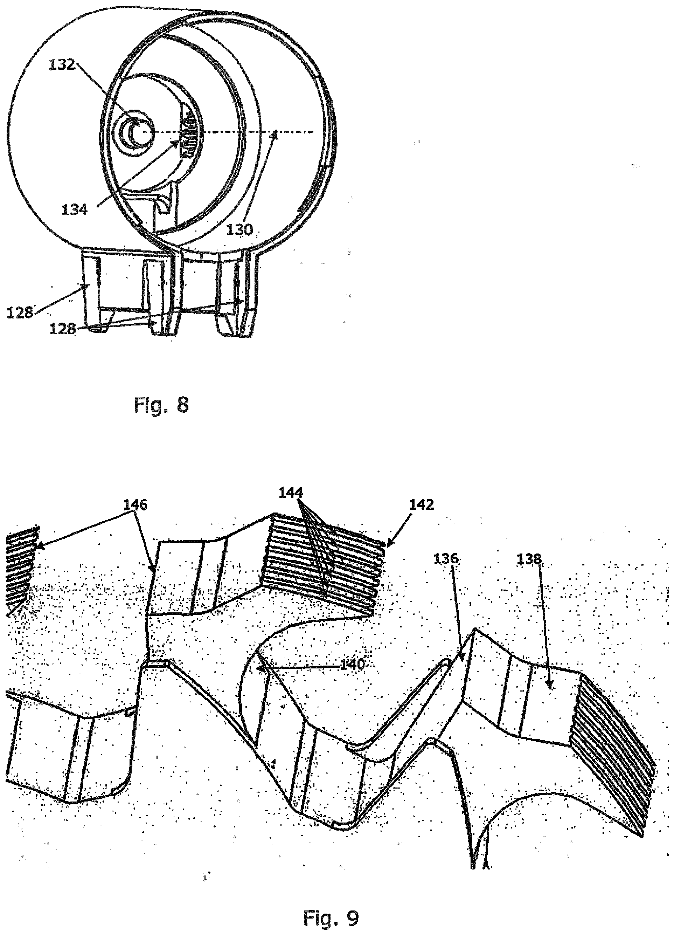

As shown in Fig. 8, the cassette housing is removably attached to the disposable unit by means of four snap locks 128. Said housing has a central axis 130 defining the centre of rotation of the cassette 104, and hence co-inciding with central axis 126 (cf. Fig. 7). A central axle 132, aligned along the central axis 130, may provide support for the cassette 104, but cassette designs eliminating the central axle are also encompassed by this disclosure In one embodiment, a short central axle is received in a central hole in the cassette and provides support to the proximal end of the cassette, while a central snap-lock provides support for the distal end. A worm gear 134 is provided for rotating the cassette 104.

The cassette 104 has one or more tissue-collecting chambers (or tissue-retaining cavities). The chambers comprise axially aligned elongate cavities that are sunk into the outer periphery of the cylindrical cassette 104, perpendicular to the direction of rotation. The number of chambers is assumed to be between 5 and 15, but may be lower or higher. Each chamber has the following features, cf. Fig. 9:
1. A flat face 136 that is roughly perpendicular to the outer periphery.
2. Adjacent to the face 136 a small cavity 138 is provided that fits the outer contour of the cutting cannula 110. In the default position of the cassette 104, the cavity 138 is positioned above the cutting cannula 110 to support the toothed rack 112 to prevent buckling-out of the rack.
3. Opposite from and at a distance to the face 136 a second face 140 is provided that is shaped as a semi-circle. The semi-circular shape of this face creates a cavity where a tissue sample may rest after having been lifted out of the tissue-receiving container 108, as will be described below.
4. The intersection of the semicircular face with the outer periphery of the cassette comprises a wedge-shaped lifting edge 142.
5. In the outer periphery of the cassette, a number of evenly spaced grooves and lands 144 circle the entire cassette parallel to the plane of rotation. The lands are evenly spaced and are configured to mesh with the ejector pins 120 as a part of the ejection sequence. The pins 120 are configured to slide in the grooves when the cassette 104 rotates, with the aim to permit the cassette 104 to rotate while the pins 120 are fully extended. These grooves and lands impact on the shape of the lifting edge, imparting on it a shape like a fork with very broad and short teeth.
6. The gap between the flat face and the edge of the semicircular face comprises the opening 146 of each chamber, through which tissue will enter the chamber. The chambers are dimensioned to easily receive the biggest sample that may be produced by a tissue-receiving container of a given size.

The nature and configuration of these features is illustrated in Fig. 9.

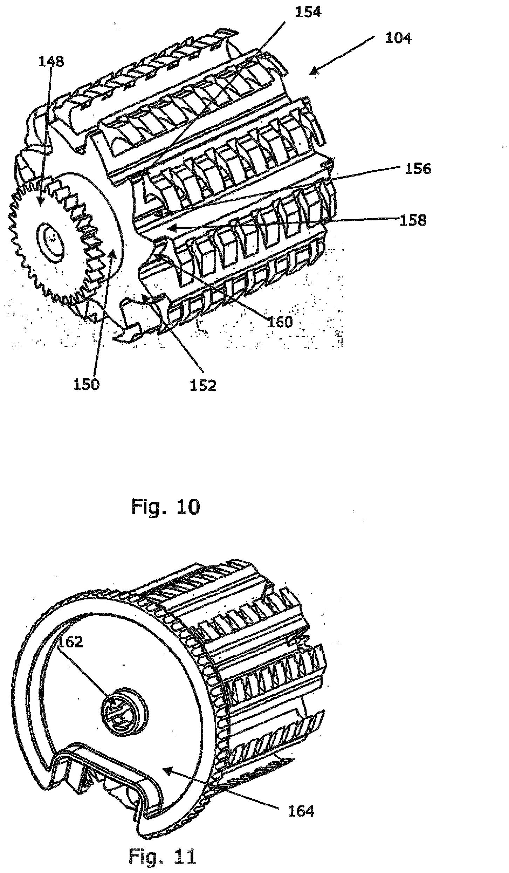

In addition to these features, the cassette cylinder has a flattened proximal end and a flattened distal end that are parallel to the plane of rotation. These ends are discussed further below with reference to Fig. 10.

Referring to Fig. 10, the proximal end provides a mechanical interface that may be in operative connection with a power transmission mechanism to allow the stepwise rotation of the cassette about its central axis. The direction of rotation is clockwise, when the cassette is viewed from the proximal end towards the distal end.

In one embodiment, the mechanical Interface to the power transmission mechanism is a gear wheel 148 that is placed parallel to the plane of rotation of the cassette 104. In addition, the gearwheel is connected to the body of the cassette 104 with a spacer bushing 150. In the center of the gear wheel and the spacer bushing 150 may be provided a hole that may be configured to receive a central axle.

Further, both the proximal and the distal end of the cassette provide means of moving the ejector frame in a vertically reciprocating motion to alternately insert the ejector pins 120 into the cavity of the tissue-receiving container 104 to eject a tissue tissue sample and retract them from said cavity when a tissue sample has been ejected. In one embodiment, the means of moving the ejector frame are twin actuator ridges 152 roughly resembling the blade of a circular saw that are mounted on the proximal and distal ends of the cassette body parallel to the plane of rotation. The actuator ridges 152 comprise ridge tops 154, ridge flanks 158, ridge bottoms 156, and ridge ramps 160.

In one embodiment the reciprocating motion of the ejector frame is stepwise to allow time for the device to carry out other functions. Such stepwise motion may be accomplished through suitable design of the actuator ridges and/or through alternate pausing and starting of the rotation of the cassette.

As shown in Fig. 11, the distal end of the cassette features a central snap-lock 162 that is rotatably received In a central hole in a lid-like portion 164 of the cassette housing and acts as a cassette support axle. Said lid-like portion is configured to be removed from the cassette housing following a biopsy procedure to provide access to the tissue samples. The snap-lock ensures that the cassette stays attached to the lid-like portion during removal and further acts as a gripping surface permitting the operator to handle the cassette without touching the samples. In addition, the snap-lock permits the cassette to be removed from the lid-like portion if the operator desires to separate these two items.

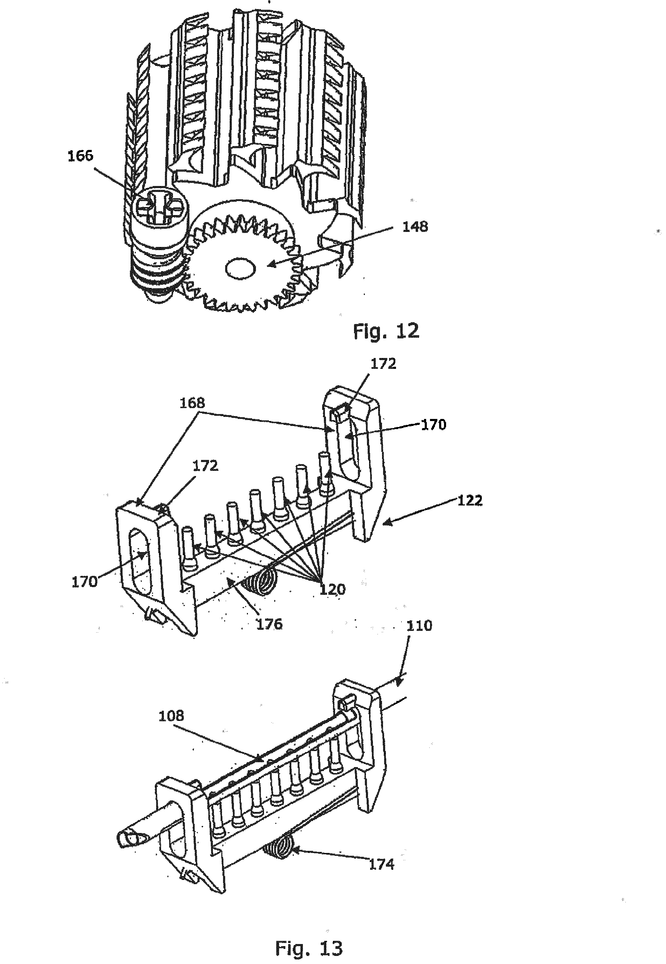

Rotation of the cassette is driven by a motor that is controlled to provide step-wise rotation at predetermined intervals. This drive system will be described further below with reference to Fig. 12.

Referring to Fig. 12, the gear wheel of the cassette is in operative connection with a worm gear 166 that is vertically mounted in the housing of the cassette and has an upper and a lower end. The upper end comprises a screw slot with a cross-point configuration that may receive a motor axle in operative engagement with the cassette motor. The lower end is rotatably lodged in a recess in the housing of the cassette.

To provide the operator of the device with the option of harvesting multiple samples in a single device insertion, samples that are held in the tissue-receiving container following a biopsy will have to be removed from said container. Removal is accomplished by means of a vertically movable ejector frame that is in operative engagement with the cassette, cf. the below description of Fig. 13.

As shown in Fig. 13, the ejector frame 122 comprises two vertical arms 168, each with a motion guide slot 170 that is shaped to receive the outer cutting cannula 110. Each arm 168 further comprises an actuator pin 172 that is configured to interface with one of the actuator ridges of the cassette 104. Thus, rotation of the cassette 104 may be translated to vertical motion of the ejector frame 122 between a lower default position and an upper elevated position. Motion from the lower default position to the upper elevated position is driven by a wire spring 174 that is placed below the ejector frame 122, while motion from the upper elevated position to the lower default position is driven by the teeth of the actuator ridges pressing down on the actuator pins 172.

Between the two arms 168 is placed a middle section 176 with a length that is slightly larger than the length of the cylinder and which comprises a number of vertical ejector pins 120. Each of these ejector pins is configured so as to fit in a corresponding ejector pin hole in the bottom of the cutting cannula 110, and a corresponding ejector pin hole in the bottom of the tissue-receiving container 108.

To provide the operator with the option of visually inspecting a harvested tissue sample following ejection without having to interrupt the biopsy procedure to remove the sample from the biopsy device, an ocular, a periscopic cone, a camera or another means of gathering an image of the tissue sample from one location and projecting it to another location immediately following the placement of the tissue-sample in the tissue-collecting cassette, is placed in visually unobstructed communication with the cassette.

In one embodiment of the device, a periscopic cone that is made of acryl or a material with similar light-conducting properties is placed immediately adjacent to the point where the cassette meets the tissue-receiving container to collect a tissue sample. Thus, the tissue sample may be visually inspected immediately after being ejected from the tissue-receiving container and the operator may inspect the harvested tissue sample to assess its adequacy.

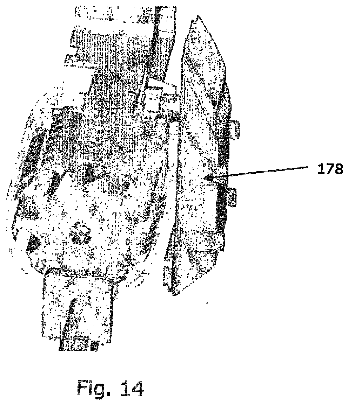

The periscopic cone 178 illustrated in Fig. 14 preferably comprises a vertical wand of acryl or a similar optically conductive material that constitutes an optical connection between a glass plate placed immediately adjacent to the bottom of the cassette and an ocular placed in a device shell that constitutes the outer surface of the biopsy device. The wand has a width that is substantially equal to the depth of the cassette and features a mirror-like surface immediately adjacent to the glass plate and angled relative to this glass plate in a 45-degree angle. Said mirror-like surface is responsible for projecting the image from the glass plate to the ocular.

The ocular may or may not comprise means of magnifying the tissue sample to provide the operator with enhanced visualization. Such means may constitute a convex lens, an arcuate surface or a similar means of magnification. The cassette may or may not comprise a light source to provide the operator with enhanced visualization.

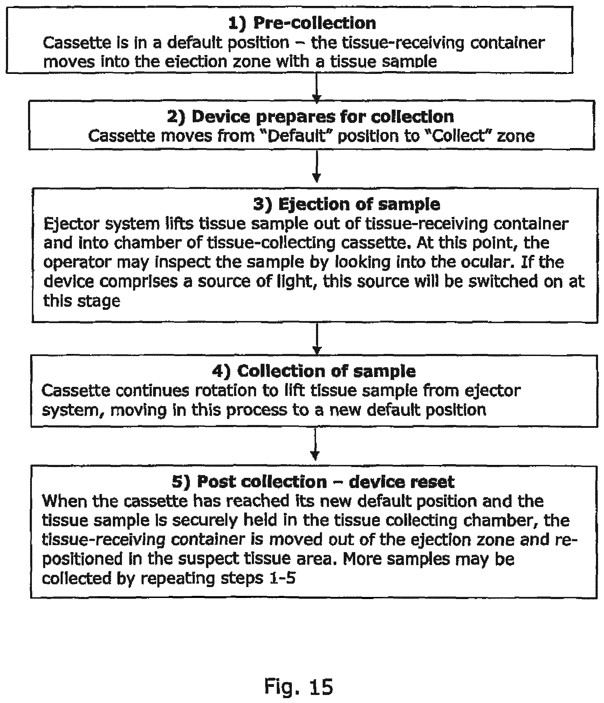

The generic operation sequence of the above described tissue sample cassette is depicted in Fig. 15. A more thorough description of the operation sequence is provided below.

An operation sequence of the embodiment of Figs. 1-15 is described below:

### 1. Pre-collection

Prior to a biopsy sequence, the device is assembled and the invasive components (cutting cannula and tissue-receiving container) are inserted into the body of the patient and positioned at the target region inside the patient's body.

In a biopsy sequence, a tissue sample is harvested at the target region, whereupon it is held in the tissue-receiving container. The toothed rack is subsequently engaged to transport the tissue-receiving container containing the sample from this point to the point of collection - corresponding with the position of the tissue-collecting cassette. During transport of the tissue sample, the cassette is held in a default position. When the cassette is in such a default position, it is positioned in such a way that the cavity adjacent to the flat face of a given tissue-collecting chamber partially covers the ejector opening in the outer cutting cannula. This is done to prevent the toothed rack from buckling during advancement of the tissue-receiving container.

Further, the cassette is vertically aligned with the second retracted position of the tissue-receiving container in such a way that the circular path of a given lifting edge intercepts a point just above the edges of the lateral opening of the cutting cannula during rotation of the cassette. Thus, a given lifting edge may partially enter the lumen of the cutting cannula and the cavity of the tissue-receiving container at predetermined intervals when the cassette is rotated. Said intervals are variable according to the distance between tissue-collecting chambers and to the speed of rotation of the cassette. For safety purposes, the biopsy device is constructed in such a way that the cassette may only start rotating when the tissue-receiving container has been moved to its second retracted position.

In the default position, no part of the cassette enters the lumen of the cutting cannula or the cavity of the tissue-receiving container, and both cutting cannula and tissue-receiving container are free to move. In this way, the tissue-receiving container may be moved between its first advanced and its second retracted position without interference from the cassette.

### 2. Device prepares for tissue sample collection

Following proper positioning of the tissue-receiving container at the point of collection, the worm gear may be engaged by the handle unit to produce rotational motion of the gear wheel and thus the cassette. In rotation, the cavity adjacent to the flat face of a given chamber is moved away from its position immediately above the tissue-receiving container. This causes the gap comprising the opening of a given tissue-collecting chamber to present itself in a position immediately above the tissue-receiving container to receive a tissue sample.

### 3. Ejection of tissue sample

One or more ejector pin holes are placed in the bottom of the tissue-receiving container. These holes match a similar number of holes in the cutting cannula and when the tissue-receiving container has been correctly positioned in its second retracted position, the holes in the tissue-receiving container are aligned with the holes in the cutting cannula.

The ejector pin holes are configured to receive one or more ejector pins that are mounted on an ejector pin frame and are vertically movable between a lower default position and an upper extended position.

When in their lower default position, the ejector pins are completely retracted from the holes in the tissue-receiving container and the cutting cannula. When in their upper extended positions, the pins are fully inserted into the holes and protrude from these to a point slightly above the top of the cavity of the tissue-receiving container.

During their movement from the lower to the upper position, the ejector pins will engage the tissue sample and lift it out of the tissue-receiving container to position the tissue sample in a given gap comprising the opening of a given tissue-collecting chamber. At the top of the lifting motion, the sample is fully inserted in the gap, and the ejector pins extend to a point above the outer periphery of the cutting cannula.

The lifting and lowering motion of the ejector pins is controlled by the interplay of the cassette actuator ridges and the actuator pins of the ejector frame. When the cassette is in a default position, the actuator pins rest on the tops of the tips of a given pair of actuator ridges. When the cassette starts rotating, the actuator pins slide off the tops and start moving up the adjacent sides, and the wire spring that is positioned below the ejector frame exerts upwards pressure on the frame. Thus, the ejector frame is moved from its lower default position towards its upper extended position, intercepting and lifting the tissue sample in this movement. The speed of the upwards motion may be controlled by the speed of rotation of the cassette and/or the steepness of the ridge flanks of the actuator ridges.

As the cassette rotates, the tissue sample is moved into the line of sight of the periscopic cone. In one preferred embodiment, an LED or similar means of illumination is turned on to clearly illuminate the tissue sample. The light is kept on for a period of time sufficient to enable the operator to fully inspect the harvested tissue sample, whereupon it is turned off to save energy.

### 4. Collection of sample

When the movement of the ejector frame from the lower default position to the upper extended position has been carried out, the tissue sample is positioned in the opening gap of a tissue-collecting chamber. As will be recalled, the path of the lifting edge intercepts a point just above the edges of the lateral opening of the cutting cannula, and thus below the tissue sample when it has been maximally elevated by the ejector pins. As the rotation of the cassette continues, the grooves in the surface of the cassette mesh with the ejector pins, permitting the lifting edge to continue its movement while the ejector pins are still extended. In this way, the lifting edge may move to a point beyond the tissue sample before the top of the wedge-shaped lifting edge makes contact with the sample and lifts it off the ejector pins. Thus, the tissue sample is securely positioned in a tissue-collecting chamber.

### 5. Post collection

When the tissue sample has been lifted off the ejector pins, the cassette continues its rotation until the next chamber has been placed in its default position. During this rotation, the ejector pins are moved to their lower default position by the interplay of the actuator pins with the ridge ramps of the actuator ridges, and the ejector pins are moved out of the holes in the tissue-receiving container and the cutting cannula. When the ejector pins have moved fully out of the holes and the cassette has been positioned in a default position, the tissue-receiving container may be moved to its first advanced position, and another sampling sequence may be initiated.

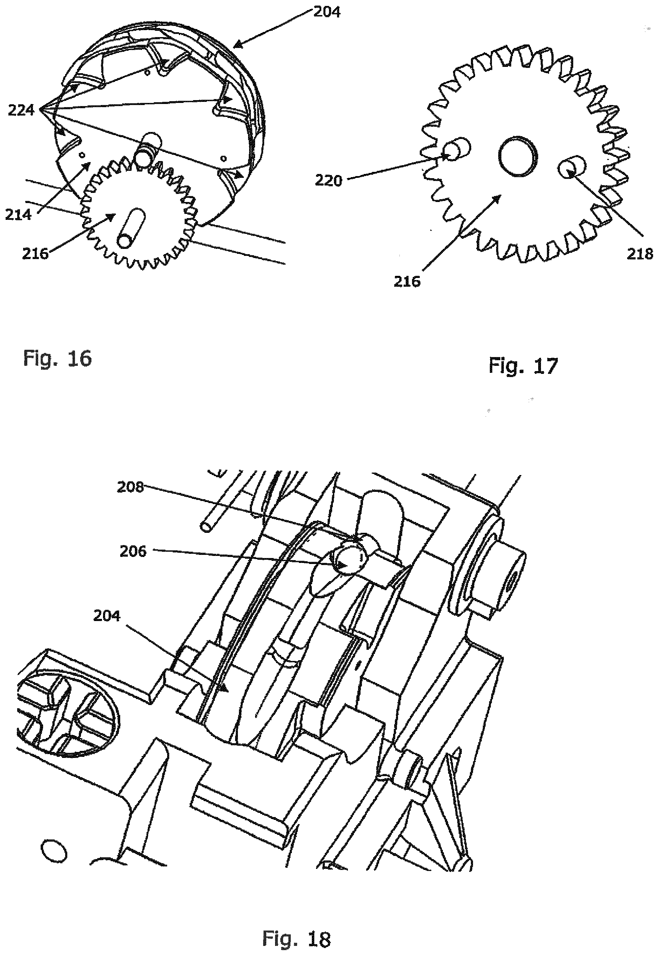

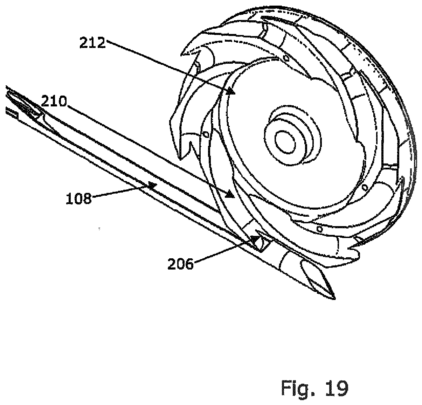

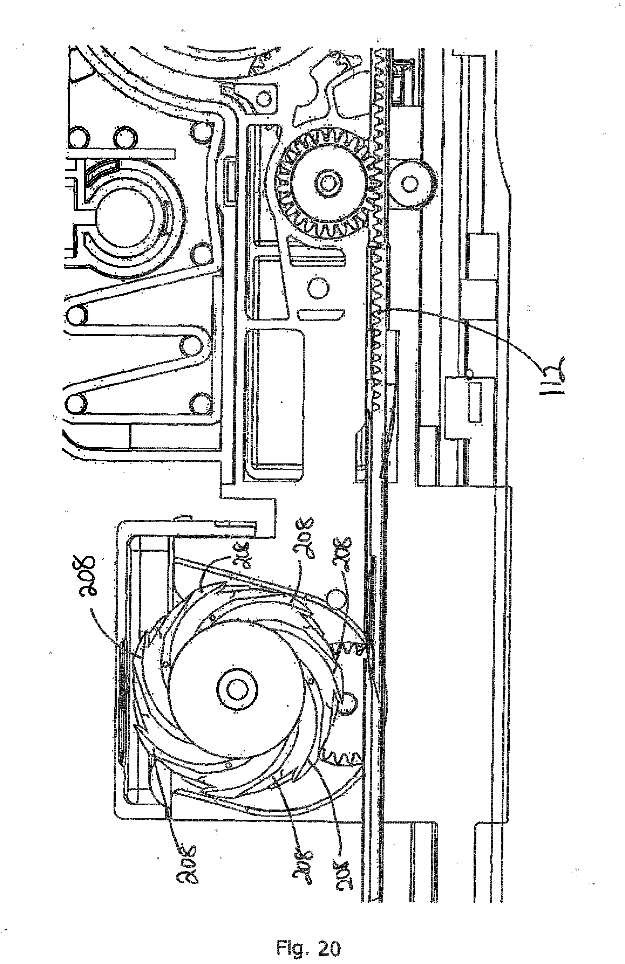

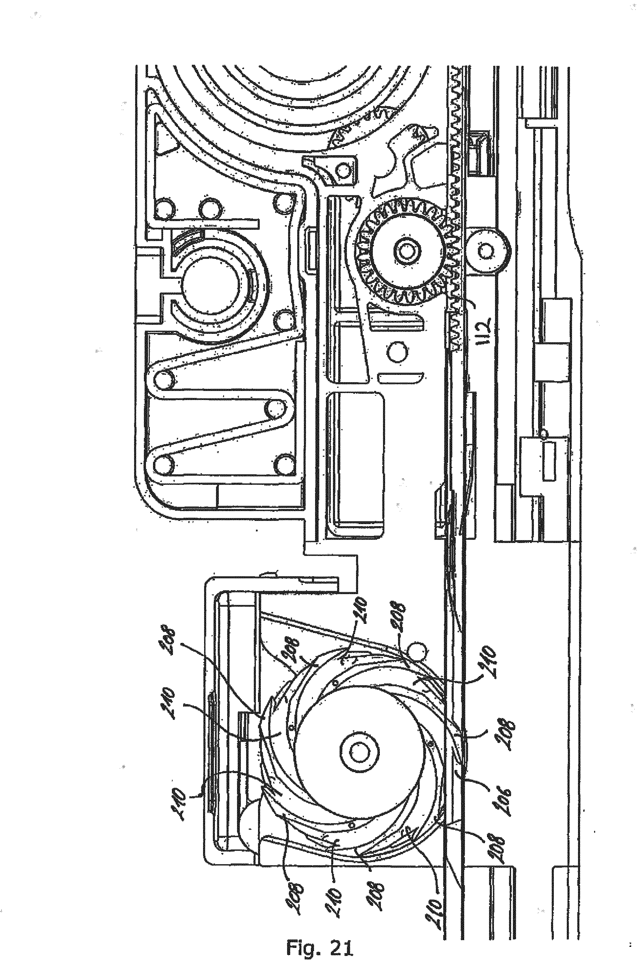

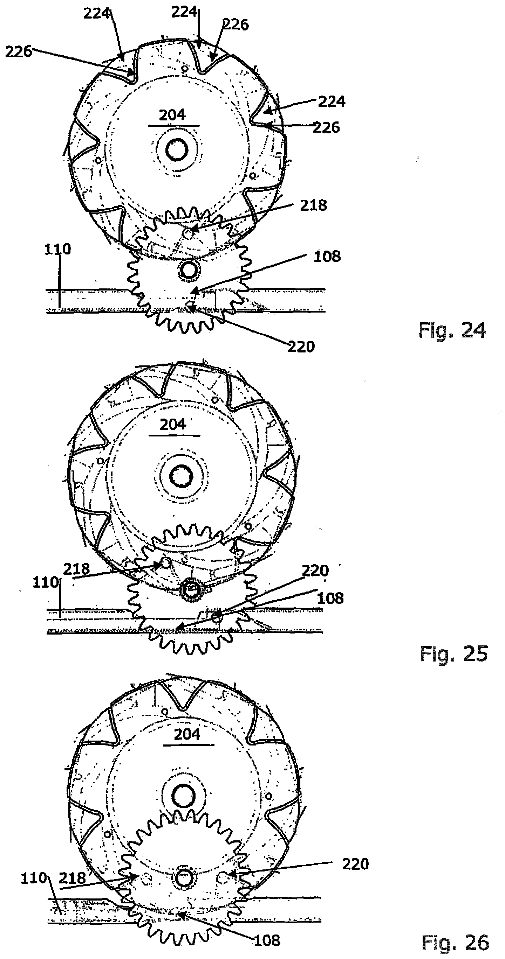

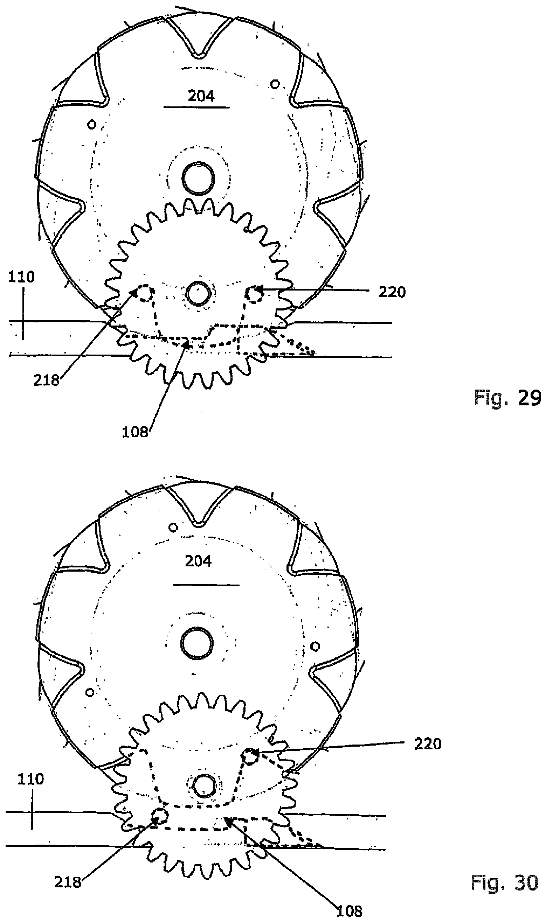

A further embodiment of a tissue-collecting system will now be described with reference to Figs 16-31. A tissue-collecting cassette 204 comprises a cassette body that comprises left and right halves. When mated, the left and right halves are configured to create a number of tissue-collecting mouths 206 (cf. Figs. 20 and 21), evenly spaced about and sunk into the outer circumference of the cassette body. Each mouth 206 is associated with a tissue-collecting rim 208 that is configured to enter the tissue-receiving cavity of the tissue-receiving container 108, and is profiled to closely fit the inner contour of tissue-receiving cavity, cf. the cross-sectional view of Figs. 20 and 21. The tissue-collecting rim 208 is herein also referred to as a scooping element. Abutting, transparent tubular sampling chambers 210 have outer openings that are in fluid communication with mouths 206 and inner openings that are in fluid communication with a central chamber 212. The central chamber 212 is herein also referred to as a mutual tissue-collecting chamber. The central chamber 212 is configured to receive an indeterminate number of tissue samples in case seven samples are deemed insufficient by the operator. The sampling chambers 210 and the central chamber 212 constitute tissue-retaining cavities.

The cassette 204 is rotatable around an axis substantially perpendicular to the longitudinal axis of the biopsy needle as discussed in more detail below. As shown in Figs. 16 and 17, the cassette is drivable via a star-shaped gear wheel 214, which in turn is driven by a two-pin driver wheel 216 having driver pins 218 and 220.

The cassette is movable between a first elevated position shown in Fig. 20 and a second lowered position shown in Fig. 21. In the second lowered position, the tissue-collecting rims 208 may engage the opening of the cutting cannula 110 to remove tissue from the cavity of the tissue-receiving container 108, as the cassette 204 is rotated in the plane of Fig. 21. The tissue-collecting mouths 206 form shoulders, which are configured to create an enclosure with the tissue-receiving cavity of the tissue-receiving container 108. This enclosure ensures that tissue held in the tissue-receiving cavity is moved into a mouth 204 and the into a sampling chamber 210.

The operational sequence of the embodiment of Figs. 16-XX is described in more detail below:

### 1. Pre-collection

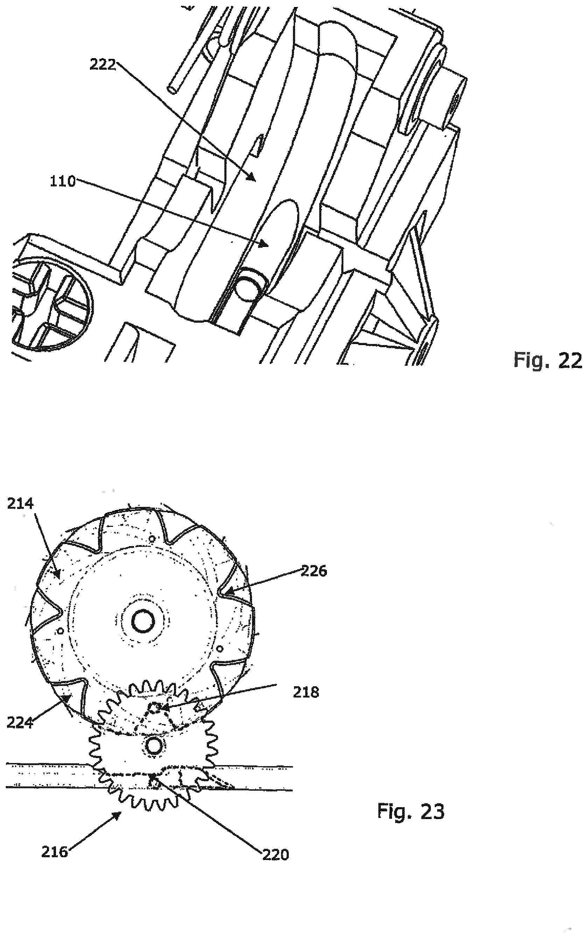

As the tissue-receiving container 204 is moved towards its second retracted position, it enters a tissue-collecting chamber 222 (cf. Fig. 22) that is a part of a disposable chassis. The motion includes three entry phases:
1. Introduction of a proximal part of the tissue-receiving container 108.
2. Introduction of a middle part of tissue-receiving container 108.
3. Introduction of a distal part of tissue-receiving container 108.

The tissue-collecting chamber 222 is formed as a cavity in disposable chassis and surrounds a proximal section of the cutting cannula 110. The lower part of the collecting chamber 222 corresponds with an upward-facing opening in the cutting cannula 110 when the cannula is positioned in its first advanced position.

The tissue-collecting cassette 204 is received in the tissue-collecting chamber 222 and is capable of reciprocating between the first elevated position (default position) and the second lowered position.

Vertical movement is the consequence of the interaction between a modified Geneva drive, comprising the two-pin drive wheel 216 (cf. Figs. 16 and 17) that is operatively connected with the star-shaped gearwheel 214 of the tissue-collecting cassette 204, and a spring (not shown), which exerts downward pressure on a central axle of the tissue-collecting cassette 1.

### 2. Device prepares for collection

A cassette driver motor (not shown) is provided for driving the tissue-collecting cassette 204. Opposing driver pins 218 and 220 of the two-pin driver wheel 216 (cf. Fig. 17) are in temporary operative engagement with the star-shaped gear wheel 214 of the tissue-collecting cassette 204, permitting the conversion of strictly rotary motion of the two-pin driver wheel 216 to rotary-reciprocating motion of tissue-collecting cassette 1.

In the first elevated default position of tissue-collecting cassette 204, driver pin 218 of the two-pin driver wheel 216 is situated in a 12 o'clock position and is supporting the weight of tissue-collecting cassette 204 and the load of the spring (not shown) by being in operative engagement with a through formed by the bottom of a V-shaped cut-out 224 of the star-shaped gear wheel 214.

### Entry phase 1

As the proximal end of tissue-receiving container 204 reaches the distal end of tissue-collecting chamber 222 in entry phase 1, a controller circuit sets the two-pin driver wheel 216 in counter-clockwise rotating motion by means of its operative engagement with the cassette driver motor. A signal to initiate this rotating motion is emitted by the controller circuit and is based on the number of pulses that has been read out on the main driver motor, and reflects the length of toothed rack 112 as determined in an initial cutting sequence.

The rotation of the two-pin driver wheel 216 changes the relative positions of driver pins 218 and 220, causing the driver pin 218 to move from its 12 o'clock position in a downwards counter-clockwise rotational motion while the other driver pin 220 is simultaneously moved away from a 6 o'clock position in an upwards counterclockwise rotational motion.

Figs. 23-27 illustrate the motion of driver pins 218 and 220 is translated to the star-shaped gear wheel 214 in the following fashion:
12-11 o'clock: Driver pin 218 remains seated in one of the throughs 226 of the V-shaped cut-out 224 and progressively lowers the tissue-collecting cassette 204 through its downwards rotational motion towards an 11 O'clock position. At the same time, the driver pin 220 moves upwards, intercepting a left crest of a neighbouring V-shaped cut-out 224 when in a 5 o'clock position. A slight clockwise rotation of tissue-collecting cassette 204 is caused by this motion.

11-9 o'clock: Driver pin 218 remains seated in the through 226 and progressively lowers the tissue-collecting cassette 204 through its downwards motion towards a 9 o'clock position. At the same time, the other driver pin 220 travels up a left shoulder of the neighbouring V-shaped cut-out 226. The 3 o'clock position of the driver pin 220 corresponds with the 9 o'clock position of driver pin 218, and when this point is reached, the downwards motion of tissue-collecting cassette 204 is halted. At this point, the weight of tissue-collecting cassette 204 and the load of spring is equally shared by the driver pins 218 and 220.

The downwards motion of the tissue-collecting cassette 204 that corresponds with the motion of driver pin 218 from the 11 o'clock to the 9 o'clock position is dimensioned to introduce a tissue-collecting rim (or scooping element) 208 of a tissue-collecting mouth 206 into the tissue-receiving cavity of the tissue-receiving container 108 during entry phase 1. This introduction is timed to correspond with the entry of an inwardly sloping face 228 of the tissue-receiving container 108 into the tissue-collecting chamber 222. Inwardly sloping face 228 is configured to have a proximal end that is flush with the inner circumference of the proximal end of tissue-receiving container 108 and a distal end that tapers Into the floor of tissue-receiving cavity. Due to downwards pressure from the spring, the tissue-collecting rim 208 is pressed against the sloping, and follows the slope of the face from the proximal toward the distal end into tissue-receiving cavity 108 during entry phase 1.

Entry phase 1 is concluded when tissue-collecting rim 208 of tissue-collecting cassette 204 reaches the bottom of the inwardly sloping face 228, corresponding with the second lower position of tissue-collecting cassette 204.

### Collection of sample

As tissue-collecting rim 208 of tissue-collecting mouth 206 moves down inwardly sloping face 228 it makes contact with tissue that is held in tissue-receiving cavity of the tissue-receiving container 108 and starts to scoop the tissue into the tissue-collecting mouth 206 and to abut the sampling chamber 222.

### Entry phase 2

During entry phase 2, the scooping of tissue into tissue-collecting mouth 206 is continued.

In this phase, the tissue-receiving container 108 continues to travel towards its second retracted position while the tissue-collecting rim 208 rests against the bottom of tissue-receiving cavity. Since a shoulder is configured to rest against the rim 208 of the opening of the tissue-receiving cavity, creating an enclosure, tissue that is held in the tissue-receiving cavity 108 is compelled to move into a tissue-collecting mouth 206.

During entry phase 2, the two-pin driver wheel 216 is held stationary to maintain the position of the tissue-collecting cassette 204.

Entry phase 2 is concluded when the tissue-collecting rim 208 reaches the outward sloping face 228 at the distal end of the tissue-receiving cavity of the tissue-receiving container 108.

### Entry phase 3

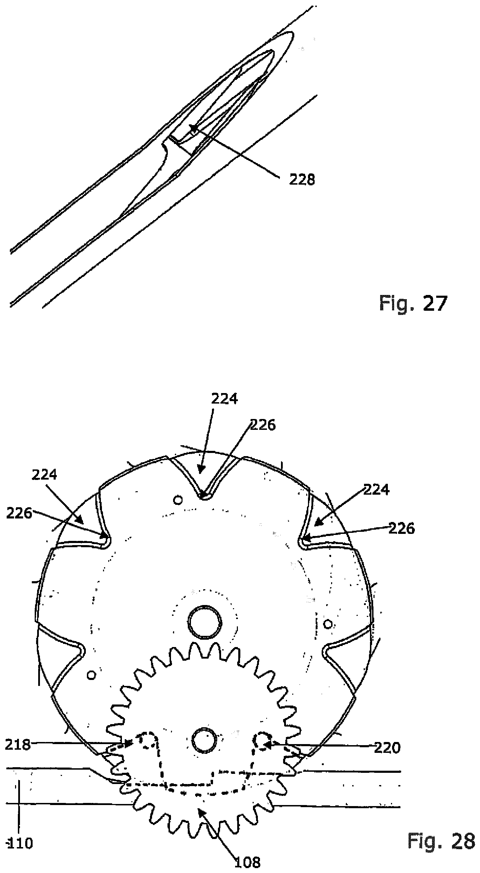

The arrival of tissue-receiving container 108 at Its utmost retracted position corresponds with the initiation of entry phase 3, as well as with the lifting of tissue-collecting cassette 204 to its first elevated default position, as illustrated In Figs. 28-31, and concludes the collection of tissue.

As the distal end of tissue-receiving container 108 reaches the distal end of tissue-collecting chamber 222 at the conclusion of entry phase 2, the controller circuit sets the two-pin driver wheel 216 in counter-clockwise rotating motion by means of its operative engagement with the cassette driver motor. The signal to initiate this rotating motion is emitted by the controller circuit and is based on the number of pulses that has been read out on main driver motor, and reflects the length of toothed rack 112 as determined in an initial cutting sequence.

The rotation of two-pin driver wheel 216 changes the relative positions of driver pins 218 and 220, causing the driver pin 218 to move from its 9 o'clock position in a downwards counter-clockwise rotational motion while the other driver pin 220 is simultaneously moved away from a 3 o'clock position In an upwards counterclockwise rotational motion.

Motion of the driver pins 218 and 220 is translated to the star-shaped gear wheel 214 In the following manner:
9-7 o'clock: Driver pin 218 is moved out of operative engagement with one of the throughs 226 and travels down a right shoulder of the associated V-shaped cut-out 224 in a counterclockwise rotational motion. At the same time, the other driver pin 220 that is seated In a neighbouring trough 226 starts moving towards a 1 o'clock position in a counterclockwise rotational motion. As driver pin 2 starts exerting upwards pressure on the tissue-collecting cassette 204 through its operative connection with the neighbouring through 226, the weight of the tissue-collecting cassette 204 and the load of spring (not shown) is transferred fully to the other driver pin 220.

In addition to the upwards motion, the driver pin 220 imparts a slight clockwise rotation on the tissue-collecting cassette 204. This rotation is calibrated to correspond with the movement of the tissue-collecting rim 208 up outwardly sloping face 228 and ensures that all remaining tissue is scooped into tissue-collecting mouth 226.

7-6 o'clock: The driver pin 216 moves off a right crest of a V-shaped cut-out 224, temporarily losing operative connection with star-shaped gear wheel 214. At the same time, the other driver pin 220 moves towards a 12 o'clock position, returning tissue-collecting cassette 204 to its first elevated default position.

A periscope (not shown) is placed immediately adjacent to the tissue-collecting cassette 204 and is configured to permit the operator to visually inspect each tissue sample following collection. For example, the periscopic cone 178 of Fig. 14 may be arranged adjacent to the tissue-collecting cassette 204. When a tissue sample has been placed in a sampling chamber 210, a small LED (not shown), which is placed adjacent to the periscope cone, is switched on to illuminate the sampling chamber with the sample. The LED 1 stays switched on for a predetermined period of for a period controlled by an operator of the biopsy device, while the device resets and prepares for the harvesting of the next tissue sample.

It has been found that the sample-collecting device (i.e. cassette) and the ejector system of Figs. 1-15 are best suitable for certain types of tissue, whereas the sample-collecting device (i.e. tissue-collecting cassette) and the ejector system of Figs. 16-31 are best suitable for other types of tissue. Generally, the setup of Figs. 1-15 is suitable for relatively firm tissue, such as cartilage and muscle tissue, whereas the setup of Figs. 16-31 is suitable for relatively soft tissue, such as breast and liver tissue.

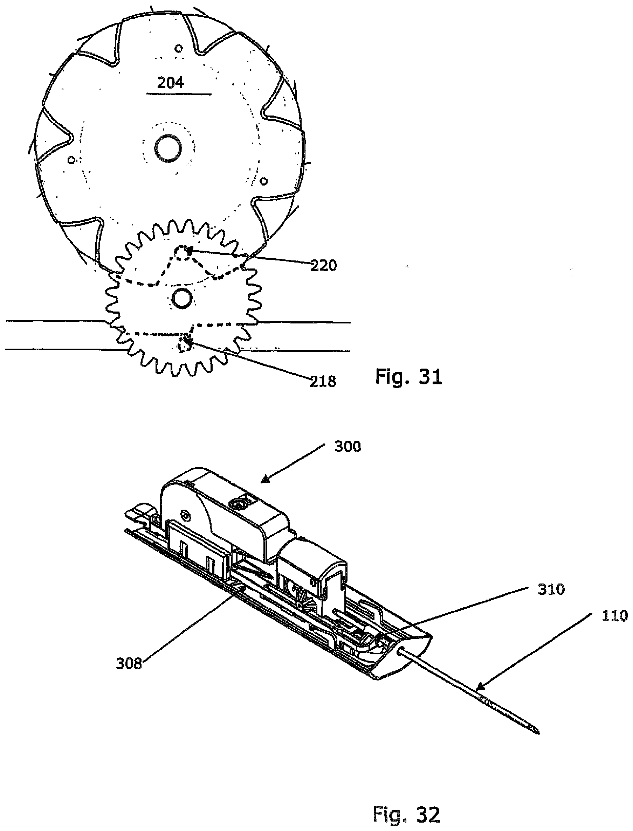

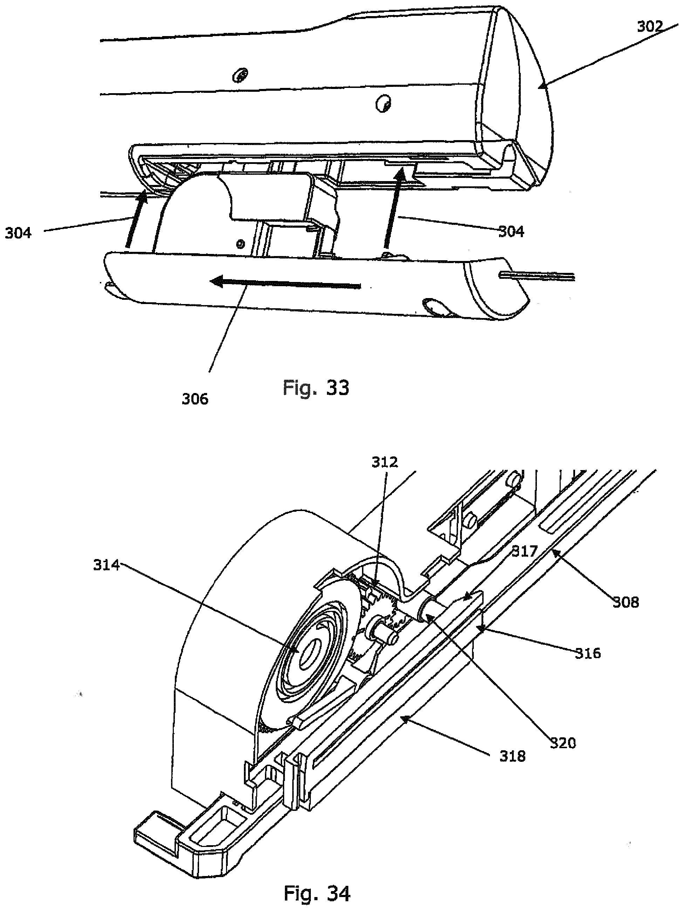

Figs. 32 and 33 generally illustrate an embodiment of a biopsy device according to the present disclosure The device comprises a disposable unit 300 (Fig. 32), which is insertable into a reusable handle unit 302 (Fig. 33). The disposable unit 300 comprises those components which come Into contact with tissue and other body matter of the patient, whereas the handle unit comprises other components, such as control circuit and motor, which do not come into contact with body matter. The disposable unit 300 is insertable into the handle unit as indicated by arrows 304 and 306 in Fig. 33. The disposable unit comprises the outer cutting cannula 110 (cf. Fig. 32) as well as the inner needle, also referred to as sample-receiving device with the tissue-receiving container 108 (not visible in Fig. 32). A needle driver 308 is provided, which is non-detachably secured to the cutting cannula 110, e.g. via an attachment element 310.

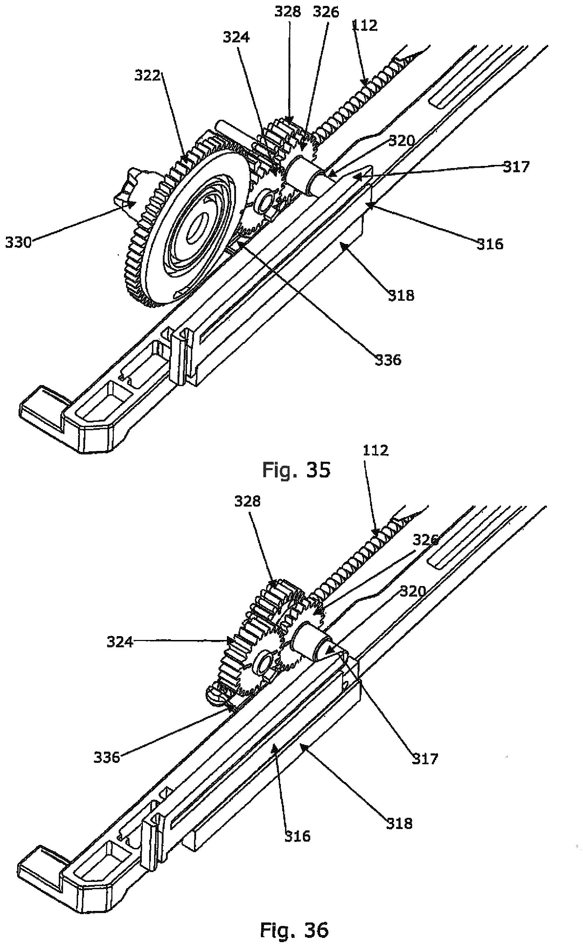

Figs. 34-38 generally illustrate an embodiment of a transport mechanism and cutting mechanism incorporated in the disposable unit 300. There is provided a drive 312 for engaging the toothed rack (elongate flexible transport element) 112, cf. e.g. Figs. 3 and 35. A colling device 314 is provided for colling up the toothed rack 112 when the sample-receiving device is moved towards its second retracted position. There is further provided an engagement slider 316 having a sloping portion 317, an engagement lift element 318, and a spring-biased connector 320. The function of these elements will be described below.

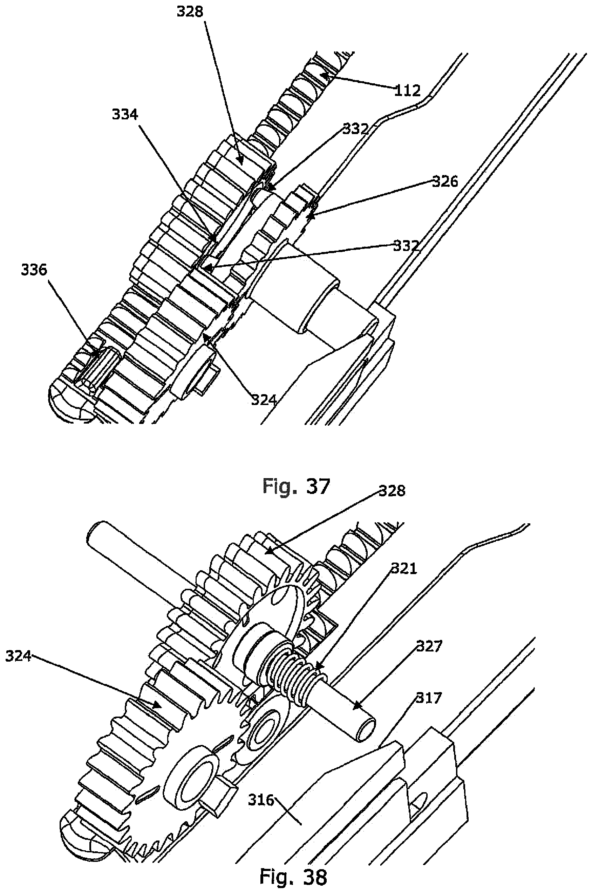

As shown in Fig. 35, the drive 312 includes four gearwheels, 322, 324, 326, and 328. A first gearwheel 322 is formed on an outer circumference of the coiling device 314, and is drivable via a connector shaft 330 for engaging a motor drive of the re-usable unit 302 (cf. Fig. 33). The first gearwheel 322 drives a second gearwheel 324, which in turn drives a third gearwheel 326, which is mounted on a common axle with a fourth gearwheel 328. In the configuration shown in Fig. 35, a side surface of the third gearwheel 326 abuts and engages a side surface of the fourth gearwheel 328. The third gearwheel 326 is transversely slidable out of engagement with the fourth gearwheel 328, cf. Fig. 37, in which the third and fourth gearwheels 326 and 328 are out of mutual engagement. As shown in Fig. 37, engagement elements 332 are provided on the third gearwheel 326 to engage corresponding engagement cavities 334 in a side surface of the fourth gearwheel 328. In both positions of the third gearwheel 326, i.e. in the position of Fig. 35 and the position of Fig. 37, its circumferential gear portion engages the second gearwheel 324. However, in the position of the gearwheels of Fig. 35, a driving force is transmittable from the connector shaft 330 to the toothed rack 112, whereas in the position of Fig. 37, the third gearwheel 326 is idling, and no driving force is transmittable to the toothed rack via the gear system described above.

The third gearwheel 326 is brought into and out of its engagement with the fourth gearwheel 328 by longitudinal displacement of the engagement slider 316 as illustrated in Figs. 35 and 36. In the advanced position of the engagement slider 316 shown in Fig. 35, the spring-biased connector 320 rests against a non-inclined surface portion of the engagement slider 316. As the engagement slider is retracted to the position shown in Fig. 36, the spring-biased connector 320 runs down the sloping surface portion 317. The connector 320 is secured to the third gearwheel, and hence an outward movement of the connector 320 also moves the third gearwheel 326 in an outward direction, i.e. out of engagement with the fourth gearwheel 328, cf. Fig. 37. The third gearwheel 326 and connector 320 are removed from the Illustration of Fig. 38 to reveal a spring 321 providing the spring-biasing force to the connector 320. Fig. 38 also illustrates a mounting axle 327 for the third and fourth gearwheels 326 and 328.

An engagement hook 336 is further provided, cf. Figs. 35, 36 and 37. The engagement hook 336 is movable between two positions. In a first position, the engagement hook is in a lowered position, in which it does not engage the toothed rack 112. The engagement hook 336 is coupled to the engagement slider 316 and the engagement lift element 318 In such a way that in the advanced position of the engagement slider shown in Figs. 34 and 35, the engagement hook is in its first position. In this position of the engagement slider 316, the third gearwheel 326 engages the fourth gearwheel 328. Consequently, movement of the toothed rack 112 and hence also of the sample-receiving device with the tissue-receiving container 108 is controlled by the drive 312, including the gearwheels 322, 324, 326 and 328. When the engagement slider 316 is retracted to the position shown in Figs. 36 and 37, the third gearwheel 326 is brought out of engagement with the fourth gearwheel 328. At the same time, due to the coupling of the hook 336 with the engagement slider 316 and the engagement lift element 318, the hook raises and engages an eye provided in a distal end portion of the toothed rack 112, as shown In Fig. 37. As the third gearwheel 326 is brought out of engagement wit the fourth gearwheel 328, movement of the toothed rack and hence also of the sample-receiving device with the tissue-receiving container 108 is no longer controlled by the drive, but by the needle driver 308, which supports the engagement hook 336.

It will hence be appreciated that in the position of Figs. 36 and 37, longitudinal displacement of the needle driver 308 causes the outer cutting cannula as well as the inner needle to displace longitudinally, as the needle driver 308 is secured to the outer needle 110 (cf. attachment element 310 of Fig. 32), and as the needle driver 308 is connected to the toothed rack 112 via the engagement hook 336. The needle driver 308 is connected to a firing mechanism including one or more springs for firing the needle driver in a forward direction to cause the outer cutting cannula as well as the inner needle (with sample-receiving device) to simultaneously advance rapidly. Such simultaneous firing of the outer and inner needles may be used to pierce a target tissue, i.e. to introduce the outer and inner needles into the target tissue mass prior to tissue harvesting,

In the position of Figs. 34 and 35, firing of the needle driver 308 merely causes firing of the outer cutting cannula 310, as the hook 336 and thus also the needle driver 308 are disengaged from the toothed rack 112 and hence from the sample-receiving device and inner needle. Accordingly, when the needle driver 308 is fired in the position of Figs. 34 and 35, the outer cutting cannula 110 may sever tissue drawn into the tissue-receiving container 108 by vacuum suction. A driving force subsequently is applied to the connector shaft 330 is transmitted via gearwheels 322, 324, 326 and 328 to the toothed rack 112, as the third and fourth gearwheels 326 and 328 are in mutual engagement as described above. Hence, the sample-receiving device may be moved between its first advanced position, in which tissue is severed, and the second retracted position, in which a tissue sample is ejected from the tissue-receiving container 108, e.g. by means of the ejector and collecting system described above with reference to Figs. 1-31.

In order to fire the outer cannula 110 and possibly also the inner needle, a spring-loaded firing system may be provided. The firing system may e.g. comprise one single spring or two separate springs, e.g as disclosed in WO : 2006/005342.

## Claims

1. A biopsy device for harvesting at least one tissue sample from a body of a living being, comprising:
a hollow needle with a distal end portion adapted to be introduced into the body;
a cutting mechanism (110) for severing the at least one tissue sample;
a tissue sample-receiving device (108) for receiving the at least one severed tissue sample, the tissue sample-receiving device being receivable in the hollow needle and movable therein between a first extended position and a second retracted position;
a tissue sample-collecting device (104, 204) comprising a rotatable drum having a plurality of tissue- retaining cavities; and
an ejector system (120, 208) for ejecting the tissue sample from the tissue sample-receiving device into one of said tissue-retaining cavities, when the tissue sample-receiving device is in the second retracted position; wherein the rotatable drum is rotatable relative to the tissue sample-receiving device in such a way that different tissue samples harvested at different times can be accommodated in separate ones of said plurality of tissue-retaining cavities, wherein the plurality of tissue-retaining cavities are arranged along a circumference of the drum, **characterised in that** said cavities are axially aligned elongated cavities that are sunk into the outer periphery of the drum, perpendicular to the direction of rotation.

2. A biopsy device according to claim 1, wherein the tissue sample-collecting device is comprised in a disposable unit of the biopsy device,

3. A biopsy device according to claim 1 or claim 2, wherein said drum (104) is rotatable around an axis of rotation, which is essentially parallel to a longitudinal axis of the hollow needle.

4. A biopsy device according to claim 1 or claim 2, wherein said drum (204) is rotatable around an axis of rotation, which extends transversely to a longitudinal axis of the hollow needle.

5. A biopsy device according to any of the preceding claims, wherein said tissue sample-collecting device comprises at least one scooping element (142, 208) arranged on its outer circumference, said scooping element being arranged to collect tissue in the tissue sample-receiving device, when the tissue sample-receiving device is in its second retracted position or when the tissue sample-receiving device is moving towards its second retracted position.

6. A biopsy device according to claim 5, wherein said tissue sample-collecting device is movable between a first elevated position, in which said scooping element does not engage a tissue-receiving container of the tissue sample-receiving device, and a second lowered position, in which said scooping element engages said tissue-receiving container.

7. A biopsy device according to claim 5 or claim 6, wherein said at least one scooping element forms at least a wall of at least one of said tissue-retaining cavities.

8. A biopsy device according to any of the preceding claims, wherein each of said tissue-retaining cavities communicates with a mutual tissue-collecting chamber (212).

9. A biopsy device according to claim 8, wherein the tissue sample-collecting device and the ejector system are operable to selectively (a) collect a plurality of tissue samples in two separate tissue-retaining cavities, or (b) collect a plurality of tissue samples in the mutual tissue-collecting chamber.

10. A biopsy device according to claim 8 or 9 and one of claims 5 to 7, wherein the mutual tissue-collecting chamber is provided centrally with respect to the circumferentially arranged scooping elements.

11. A biopsy device according to any preceding claim, wherein the biopsy device further comprises an inspection system (178) arranged to allow an operator of the biopsy device to visually inspect the tissue sample ejected into said tissue-retaining cavity, while said hollow needle remains in the body.

12. A biopsy device according to claim 11, wherein the inspection system comprises at least one of: an ocular, a periscopic cone, and a camera system.

13. A biopsy device according to claim 11, in which the inspection system is arranged such that it allows for inspection of a tissue sample after ejection of the tissue sample into one of said cavities and after relative movement of the tissue sample-receiving device and the tissue sample-collecting device to relatively displace said one cavity accommodating the tissue sample away from that position, in which the tissue sample has been ejected into said one cavity.

## Patentansprüche

1. Biopsievorrichtung zur Gewinnung mindestens einer Gewebeprobe aus einem Körper eines Lebewesens, umfassend:
eine Hohlnadel mit einem distalen Endabschnitt, der angepasst ist, um in den Körper eingeführt zu werden;
einen Schneidmechanismus (110) zum Abtrennen der mindestens einen Gewebeprobe;
eine Gewebeproben-Aufnahmevorrichtung (108) zur Aufnahme der mindestens einen abgetrennten Gewebeprobe, wobei die Gewebeproben-Aufnahmevorrichtung in der Hohlnadel aufgenommen und darin zwischen einer ersten ausgefahrenen Position und einer zweiten eingezogenen Position bewegt werden kann;
eine Gewebeproben-Sammelvorrichtung (104, 204), umfassend eine drehbare Trommel, die eine Vielzahl von Gewebehaltehohlräumen aufweist; und
ein Ausstoßersystem (120, 208) zum Ausstoßen der Gewebeprobe aus der Gewebeproben-Aufnahmevorrichtung in einen der Gewebehaltehohlräume, wenn die Gewebeproben-Aufnahmevorrichtung in der zweiten eingezogenen Position ist; wobei die drehbare Trommel relativ zu der Gewebeproben-Aufnahmevorrichtung derart drehbar ist, dass verschiedene Gewebeproben, die zu verschiedenen Zeiten gewonnen werden, in getrennten der Vielzahl von Gewebehaltehohlräumen untergebracht werden können,
wobei die Vielzahl von Gewebehaltehohlräumen entlang eines Umfangs der Trommel angeordnet sind, **dadurch gekennzeichnet, dass** die Hohlräume axial ausgerichtete längliche Hohlräume sind, die senkrecht zur Drehrichtung in den Außenumfang der Trommel versenkt sind.

2. Biopsievorrichtung nach Anspruch 1, wobei die Gewebeproben-Sammelvorrichtung in einer Einwegeinheit der Biopsievorrichtung enthalten ist.

3. Biopsievorrichtung nach Anspruch 1 oder Anspruch 2, wobei die Trommel (104) um eine Drehachse drehbar ist, die im Wesentlichen parallel zu einer Längsachse der Hohlnadel ist.

4. Biopsievorrichtung nach Anspruch 1 oder Anspruch 2, wobei die Trommel (204) um eine Drehachse drehbar ist, die sich quer zu einer Längsachse der Hohlnadel erstreckt.

5. Biopsievorrichtung nach einem der vorstehenden Ansprüche, wobei die Gewebeproben-Sammelvorrichtung mindestens ein Schöpfelement (142, 208) umfasst, das an ihrem äußeren Umfang angeordnet ist, wobei das Schöpfelement angepasst ist, um Gewebe in der Gewebeproben-Aufnahmevorrichtung zu sammeln, wenn die Gewebeproben-Aufnahmevorrichtung in ihrer zweiten eingezogenen Position ist oder wenn sich die Gewebeproben-Aufnahmevorrichtung zu ihrer zweiten eingezogenen Position hin bewegt.

6. Biopsievorrichtung nach Anspruch 5, wobei die Gewebeproben-Sammelvorrichtung zwischen einer ersten, angehobenen Position, in der das Schöpfelement einen Gewebeaufnahmebehälter der Gewebeproben-Aufnahmevorrichtung nicht in Eingriff nimmt, und einer zweiten, abgesenkten Position, in der das Schöpfelement den Gewebeaufnahmebehälter in Eingriff nimmt, beweglich ist.

7. Biopsievorrichtung nach Anspruch 5 oder Anspruch 6, wobei das mindestens eine Schöpfelement mindestens eine Wand mindestens eines der Gewebehaltehohlräume bildet.

8. Biopsievorrichtung nach einem der vorstehenden Ansprüche, wobei jeder der Gewebehaltehohlräume mit einer gemeinsamen Gewebesammelkammer (212) in Verbindung steht.

9. Biopsievorrichtung nach Anspruch 8, wobei die Gewebeproben-Sammelvorrichtung und das Ausstoßersystem so betrieben werden können, dass sie selektiv (a) eine Vielzahl von Gewebeproben in zwei getrennten Gewebehaltehohlräumen sammeln oder (b) eine Vielzahl von Gewebeproben in der gemeinsamen Gewebesammelkammer sammeln.

10. Biopsievorrichtung nach Anspruch 8 oder 9 und nach einem der Ansprüche 5 bis 7, wobei die gemeinsame Gewebesammelkammer zentral in Bezug auf die in Umfangsrichtung angeordneten Schöpfelemente vorgesehen ist.

11. Biopsievorrichtung nach einem der vorstehenden Ansprüche, wobei die Biopsievorrichtung weiter ein Inspektionssystem (178) umfasst, das so angeordnet ist, dass ein Bediener der Biopsievorrichtung die Gewebeprobe, die in den Gewebehaltehohlraum ausgestoßen wird, visuell inspizieren kann, während die Hohlnadel im Körper verbleibt.

12. Biopsievorrichtung nach Anspruch 11, wobei das Inspektionssystem mindestens eines umfasst von: einem Okular, einem Periskopkegel und einem Kamerasystem.

13. Biopsievorrichtung nach Anspruch 11, bei der das Inspektionssystem so angeordnet ist, dass es eine Inspektion einer Gewebeprobe nach Ausstoß der Gewebeprobe in einen der Hohlräume und nach Relativbewegung der Gewebeproben-Aufnahmevorrichtung und der Gewebeproben-Sammelvorrichtung zum relativen Verschieben des einen Hohlraums, in dem die Gewebeprobe untergebracht ist, von der Position weg, in der die Gewebeprobe in den einen Hohlraum ausgestoßen worden ist, gestattet.

## Revendications

1. Dispositif de biopsie destiné à récolter au moins un échantillon de tissu à partir du corps d'un être vivant, comprenant :
une aiguille creuse munie d'une partie d'extrémité distale conçue pour être introduite dans le corps ;
un mécanisme de coupe (110) pour découper l'au moins un échantillon de tissu ;
un dispositif de réception d'échantillon de tissu (108) destiné à recevoir l'au moins un échantillon de tissu découpé, le dispositif de réception d'échantillon de tissu pouvant être reçu dans l'aiguille creuse et pouvant se déplacer à l'intérieur de celle-ci entre une première position étendue et une seconde position rétractée ;
un dispositif de collecte d'échantillon de tissu (104, 204) comprenant un tambour rotatif présentant une pluralité de cavités de retenue de tissu ; et
un système d'éjection (120, 208) destiné à éjecter l'échantillon de tissu du dispositif de réception d'échantillon de tissu dans l'une desdites cavités de retenue de tissu, lorsque le dispositif de réception d'échantillon de tissu se trouve dans la seconde position rétractée ; dans lequel le tambour rotatif peut pivoter par rapport au dispositif de réception d'échantillon de tissu de telle manière que les différents échantillons de tissu récoltés à différents moments peuvent être logés dans des cavités séparées de ladite pluralité de cavités de retenue de tissu,
dans lequel la pluralité de cavités de retenue de tissu est agencée le long d'une circonférence du tambour, **caractérisé en ce que** lesdites cavités sont des cavités de forme allongée alignées de manière axiale qui sont enfoncées dans la périphérie externe du tambour, perpendiculaires au sens de rotation.

2. Dispositif de biopsie selon la revendication 1, dans lequel le dispositif de collecte d'échantillon de tissu est compris dans une unité jetable du dispositif de biopsie.

3. Dispositif de biopsie selon la revendication 1 ou la revendication 2, dans lequel ledit tambour (104) peut pivoter autour d'un axe de rotation, qui est sensiblement parallèle à un axe longitudinal de l'aiguille creuse.

4. Dispositif de biopsie selon la revendication 1 ou la revendication 2, dans lequel ledit tambour (204) peut pivoter autour d'un axe de rotation, qui s'étend de manière transversale à un axe longitudinal de l'aiguille creuse.

5. Dispositif de biopsie selon l'une quelconque des revendications précédentes, dans lequel ledit dispositif de collecte d'échantillon de tissu comprend au moins un élément de prélèvement (142, 208) agencé sur sa circonférence extérieure, ledit élément de prélèvement étant agencé de manière à collecter un tissu dans le dispositif de réception d'échantillon de tissu, lorsque le dispositif de réception d'échantillon de tissu se trouve dans sa seconde position rétractée ou lorsque le dispositif de réception d'échantillon de tissu est dirigé vers sa seconde position rétractée.

6. Dispositif de biopsie selon la revendication 5, dans lequel le dispositif de collecte d'échantillon de tissu peut se déplacer entre une première position élevée, dans laquelle ledit élément de prélèvement n'entre pas en prise avec un récipient de réception de tissu du dispositif de réception d'échantillon de tissu, et une seconde position abaissée, dans laquelle ledit élément de prélèvement entre en prise avec ledit récipient de réception de tissu.

7. Dispositif de biopsie selon la revendication 5 ou la revendication 6, dans lequel ledit au moins un élément de prélèvement forme au moins une paroi d'au moins une desdites cavités de retenue de tissu.

8. Dispositif de biopsie selon l'une quelconque des revendications précédentes, dans lequel chacune desdites cavités de retenue de tissu communique avec une chambre de collecte de tissu commune (212).

9. Dispositif de biopsie selon la revendication 8, dans lequel le dispositif de collecte d'échantillon de tissu et le système d'éjection peuvent être actionnés pour (a) collecter de manière sélective une pluralité d'échantillons de tissu dans deux cavités de retenue de tissu séparées, ou (b) collecter une pluralité d'échantillons de tissu dans la chambre de collecte de tissu commune.

10. Dispositif de biopsie selon la revendication 8 ou 9 et l'une quelconque des revendications 5 à 7, dans lequel la chambre de collecte de tissu commune est prévue au centre par rapport aux éléments de prélèvement agencés de manière circonférentielle.

11. Dispositif de biopsie selon l'une quelconque des revendications précédentes, dans lequel le dispositif de biopsie comprend en outre un système d'inspection (178) agencé de manière à permettre à un opérateur du dispositif de biopsie d'inspecter visuellement l'échantillon de tissu éjecté dans ladite cavité de retenue de tissu, tandis que ladite aiguille creuse reste dans le corps.

12. Dispositif de biopsie selon la revendication 11, dans lequel le système d'inspection comprend au moins un élément parmi : un système oculaire, un système à cône périscopique et un système de caméra.

13. Dispositif de biopsie selon la revendication 11, dans lequel le système d'inspection est agencé de sorte qu'il permet l'inspection d'un échantillon de tissu après éjection de l'échantillon de tissu dans l'une desdites cavités et après le déplacement relatif du dispositif de réception d'échantillon de tissu et du dispositif de collecte d'échantillon de tissu afin de déplacer de manière relative ladite une cavité logeant l'échantillon de tissu à distance de cette position, dans laquelle l'échantillon de tissu a été éjecté dans ladite une cavité.
